# EUROPEAN PATENT APPLICATION

(11) **EP 3 135 295 A1**
(43) Date of publication of application: **01.03.2017**
(21) Application number: 15182845.6
(22) Date of filing: 28.08.2015
(51) Int. Cl.: A61K 39/02, C07K 14/20

(54) **COUPLING OF BORRELIAL PROTEIN BB0689 TO VIRUS-LIKE PARTICLES FOR GENERATION OF LYME DISEASE VACCINE**

(71) Applicant: Latvian Biomedical Research and Study Centre, 1067 Riga (LV)
(72) Inventor: Tärs, Kaspars, LV-1067 Riga (LV); Petrovskis, Ivars, LV-1067 Riga (LV); Dislers, Andris, LV-1067 Riga (LV); Skrastina, Dace, LV-1067 Riga (LV); Zeltins, Andris, LV-1067 Riga (LV); Brangulis, Kalvis, LV-1067 Riga (LV); Kotelovica, Svetlana, LV-1067 Riga (LV)
(74) Representative: Zaboliene, Reda

(57) **Abstract**

An invention discloses Lyme disease vaccine by obtaining high immune response against surface protein BB069 from Borrelia burgdorferi, the causative agent of Lyme disease.

Present invention includes a protein comprising amino acids identical at least 80% to modified Borrelia burgdorferi BB0689 protein (as set forth in SEQ ID 5), chemically coupled to bacteriophage Qβ virus-like particles. Said invention also relates with a method of production and purification of protein, comprising amino acids identical at least 80% to modified Borrelia burgdorferi BB0689 protein as set forth in SEQ ID 5.

## Description

### TECHNICAL FIELD

The invention relates to method of obtaining high immune response against surface protein BB0689 from Borrelia burgdorferi, the causative agent of Lyme disease. The invention may be used to develop a vaccine against Lyme disease.

### BACKGROUND OF INVENTION

### Lyme disease

Lyme disease is the most prevalent zoonotic disease in the Northern hemisphere, caused by *Borrelia burgdorferi* sensu lato family bacteria, including *Borrelia burgorferi* sensu stricto, *Borrelia afzelii* and *Borrelia garinii.* Annualy, in Europe about 85,000 cases are reported, in United States - about 30,000 cases and it is estimated that the number of unreported cases may be up to 10 times higher [1]. Lyme disease is transmitted by infected ticks belonging to *Ixodes* genus upon blood meal. Early symptoms, which include circular area of redness around the site of tick bite, fever and headache can go unnoticed, but at later stages several complications occur, including pain in joints, severe headaches, heart palpitations, neurological disorders, memory problems and feeling constantly tired. Symptoms may persist for years, even after intense treatment with antibiotics [2].

Borrelia spirochete has adopted to persist within both tick vector and warm-blooded mammalian host. Borrelia species have many surface proteins, expression of which is frequently regulated upon transfer from tick to mammals as a response to dramatic change in environment. Surface proteins of Borrelia bacteria can be considered as attractive target for vaccination. However, currently there is no vaccine available at market. SmithKline Beecham (now GlaxoSmithKline) LYMErix vaccine was introduced in 1998, but removed from market due to complaints about arthritis, caused by vaccination [3]. LYMErix was based on recombinant Borrelia burgdorferi surface protein OspA. OspA has a stretch of homology to human leukocyte function-associated antigen-1 (hLFA-1), therefore an autoimmune response to self-antigen may occur upon vaccination. Also, LYMErix was efficient only against *Borrelia burgdorferi* sensu stricto strain, found predominantly in North America and not in Europe. In later years there have been several attempts to generate other vaccine candidates, but essentially all of them have been variants of LYMErix based on OspA and none of them has reached the market [4]. Therefore, there is a need for another Lyme disease vaccine, preferably based on proteins, other than OspA [5].

### Protein BB0689

BB0689 is borrelial outer surface protein [6] with a largely unknown function. Expression of BB0689 is upregulated upon transfer of bacteria to mammalian host. BB0689 protein is well conserved across various Borrelia species, proteins of *Borrelia burgdorferi* sensu stricto, *Borrelia garinii* and *Borrelia afzelii* displaying about 87% sequence identity. Antibodies against BB0689 have been demonstrated to have bactericidal properties [6]. Like most borrelial surfce proteins, BB0689 has an N-terminal signal sequence, necessary for insertion in the membrane.

### VLPs as adjuvants

Virus-like particles (VLPs), essentially empty shells of viruses, obtained by recombinant techniques, are suitable carriers for foreign antigens and they are known to substantially enhance the immune response and protectivity compared to antigens alone. Foreign epitopes on the surface of VLPs are arranged in a highly regular and repetitive manner, which is efficiently recognized by the immune system as a trait of pathogens. This phenomenon can be explained at a molecular level - repetitive structures are able to crosslink B cell receptors (e.g. immunoglobulins) on the surface of B lymphocytes, which is a potent signal for B-cell activation (see [7] for a review).

VLPs of RNA bacteriophages, including MS2, Qβ, AP205 and PP7 have been extensively used as versatile carriers of foreign epitopes.

Patent CS19920003779 discloses Bivalent immunogens effective against diseases caused by viruses from the Orthomyxoviridae or Paramyxo-viridae tribe and gram-negative bacteria or spirochete Borrelia Burgdorferi consist of virus glycoproteins connected via their C- or N- endings with bacterial (spirochete) proteoses. The immunogens are prepared by a compound dialysis containing a detergent, virus glycoproteins and bacterial (spirochete) proteoses with a physiological medium at progressive pH decrease. After detergent's disposal, there is a mutual bonding between virus and bacterial molecules. Purified preparation is dried in lyophilisation. This is a method to generate a vaccine simultaneously against certain viruses and some bacteria.

Patent US20040935494 discloses recombinant canine herpes virus (CHV). The recombinant CHV includes and expresses at least one heterologous nucleotide sequence encoding an antigen. The antigen can be canine distemper virus HA, canine distemper virus F, rabies virus G, canine parvovirus VP2, parainfluenza virus type 2 HA, parainfluenza virus type 2 F, Borrelia *burgdorferi* OspA, or Borrelia burgdorferi OspB. The at least one heterologous nucleotide sequence can be in at least one insertion site selected from the group consisting of ORF3 (SEQ ID NO:4), ORF5 (SEQ ID NO:5), the thymidine kinase gene, and the intergenic region corresponding to genes coding for the large subunit and the small subunit. Immunological or vaccine compositions as well as methods for inducing an immunological response are also disclosed and claimed. This patent is the most related as the idea is similar - to combine borrelial proteins with virus-like particles. It also makes use of virus-like particles, albeit of different kind - those of canine herpes virus. The borrelial proteins used are OspA (Lymerix) and OspB, both very different from BB0689.

### SUMMARY OF THE INVENTION

The aim of the invention is to offer Lyme disease vaccine by obtaining high immune response against surface protein BB069 from Borrelia burgdorferi, the causative agent of Lyme disease.

The invention may be used to develop a vaccine against Lyme disease.

Present invention includes a protein comprising amino acids identical at least 80% to modified Borrelia burgdorferi BB0689 protein (as set forth in SEQ ID 5), chemically coupled to bacteriophage Qβ virus-like particles.

Said invention also relates with a method of production and purification of protein, comprising amino acids identical at least 80% to modified Borrelia *burgdorferi* BB0689 protein as set forth in SEQ ID 5.

According to another embodiment, the present invention relates to a method for chemical coupling of protein, comprising amino acids identical at least 80% to modified Borrelia *burgdorferi* BB0689 protein (as set forth in SEQ ID 5) to bacteriophage Qβ virus-like particles.

### BRIEF DESCRIPTION OF THE DRAWINGS

Fig. 1 shows coomasie stained SDS-PAGE gel, visualizing the course of BB0689 production and purification. Lane 1- E. coli culture lysate before induction, lane 2 - E. coli culture lysate 4h after induction, lane 3 - BB0689 after metal affinity purification, lane 4 - BB0689 after gel filtration purification, lane 5 - BB0689 after TEV protease cleavage, lane 6 -marker (from bottom - 14, 18, 25, 35, 45, 66,and 116 kDa).
Fig. 2 shows chemical coupling of BB0689 protein to Qβ VLPs, visualized on coomasie-stained SDS-PAGE gel. Lane 1 - Qβ, lane 2 - Qβ, treated with SULFO-KMUS linker, lane 3 - BB0689, chemically coupled to Qβ (after gel filtration), lane 4 - marker (from bottom-14, 18, 25, 35, 45, 66,and 116 kDa).
Fig. 3 shows electron micrograph of Qβ VLPs with chemically coupled BB0689 protein.

### DESCRIPTION OF PREFERRED EMBODIMENTS

### Production and purification of BB0689 protein

The coding sequence of BB0689 gene (NCBI Gene ID: 1195542), excluding signal sequence (residues 1-21) was amplified from genomic DNA of Borrelia *burgdorferi* B31 strain using the primers listed in SEQ ID No. 1 and SEQ ID No. 2.

The reverse primer contains extra codon for cysteine, which was added to c-terminus of protein to faciliate chemical coupling to VLPs. The PCR product was cleaved by NcoI and NotI and ligated into a modified pet11d vector, harboring N-terminal 6his tag and TEV protease cleavage site. The complete nucleotide sequence of plasmid is listed in SEQ ID No. 3. The resulting encoded protein sequence is shown in SEQ ID No. 4.

The plasmid was transformed into *E.coli* strain RR1 and grown overnight on agar plates, supplemented with kanamycin (50mg/l) at 37°C. Individual colonies were inoculated in 5 ml of LB media with kanamycin (50 mg/l) and cultivated overnight at 37°C. Plasmid DNA was isolated form the bacteria and sequence verified by Sanger sequencing. Plasmid was further transformed into *E.coli* strain BL21(DE3). For production of protein, cells were grown in modified 2xTY media, supplemented with kanamycin (50 mg/l), 133 mM phosphate buffer (pH 7.4) and glucose (4 g/l)) with vigorous agitation at 25°C until an OD₆₀₀ of 0.8-1.0. IPTG was then added to the final concentration of 0.2mM and grown for further 4-6 hours.

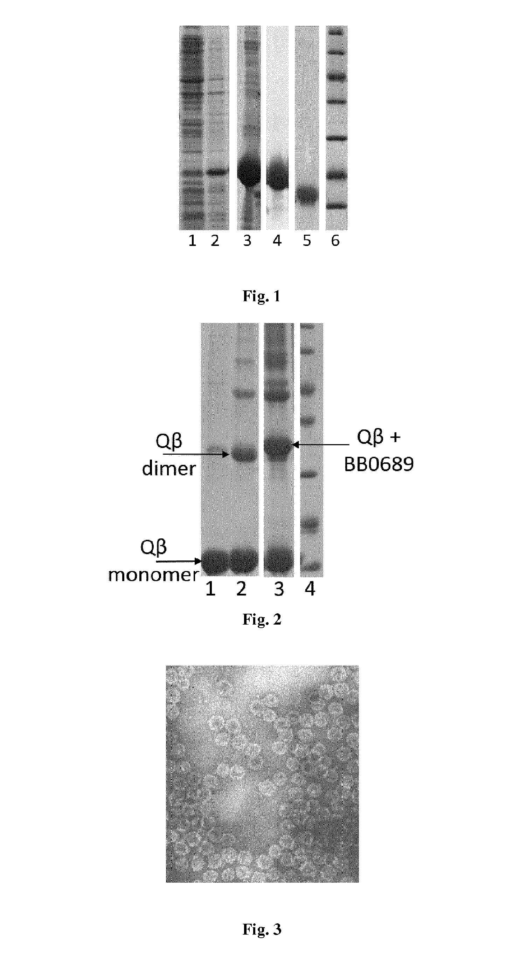

Cells were harvested by centrifugation, re-suspended in 3 volumes of buffer, containing 40 mM tris-HCl pH 8.0, 300 mM NaCl and lysed by sonication. Cell debris were removed by centrifugation. The protein was initially purified by metal affinity chromatography on 1 ml His-trap columns (GE Healthcare). Clarified lysate was passed through column with a syringe. Weakly bound cellular proteins were removed by passing 3ml of 20 mM tris-HCl, 300 mM NaCl, 20 mM imidazole. BB0689 protein was eluted with 3 ml of 20 mM tris-HCl, 300 mM NaCl, 300 mM imidazole. Protein was further applied on a 16/600 Superdex 200 column (GE Healthcare) in 20 mM tris-HCl p 8.0. Fractions, containing protein were examined on SDS-PAGE gel. BB0689 protein containing fractions were pooled, TEV protease added to a final concentration of 0.05 mg/ml and mixture incubated over night at +4°C. In order to remove cleaved His tag, remnants of uncleaved protein and TEV protease, the mixture was through 1 ml His-trap column (GE Healthcare). Protein was buffer-exchanged into 20mM HEPES pH 7.0, concentrated using 15 ml 10 kDa Amicon centrifugal filter unit (Millipore) to a concentration of 5 mg/ml and frozen at -20°C until further use. The course of BB0689 production and purification is shown in Figure 1. The amino acid sequence of cleaved BB0689 protein, used for further coupling to VLPs is shown in SEQ ID 5.

### Production and purification of bacteriophage Qβ VLPs.

Production and purification of Qβ VLPs was done as described earlier [8]. The obtained VLPs were buffer-exchanged into 20mM HEPES pH 7.0, concentrated using Amicon centrifugal filter unit (Millipore) to a concentration of 5 mg/ml and frozen at -20°C until further use.

### Chemical coupling of BB0689 protein to VLPs

To 1 ml of purified Qβ VLPs (5mg/ml) in 20 mM HEPES pH 7.0 0.5 mg SULFO-KMUS linker (Pierce) was added. The mixture was incubated for 30 minutes at room temperature. Surplus SULFO-KMUS was removed by passing through 5ml ZebaTM desalting column (Pierce). 1 ml of purified BB0689 protein (5 mg/ml) was added and mixture incubated for 30 minutes at room temperature. In order to separate VLPs with conjugated BB0689 protein from free protein, the obtained mixture was passed through a 16/600 Superdex200 column (GE Healthcare). The obtained product was examined on SDS-PAGE gel (Figure 2). The integrity of capsids was verified by electron microscopy (Figure 3).

### Immunization of mice

Pathogen-free, female BALB/c mice between 6-8 weeks of age were obtained from the Latvian Experimental Animal Laboratory of the Riga Stradins University and maintained under pathogen-free conditions in accordance with the principles and guidelines of the Latvian and European Community Laws.

Five animals were used in each experimental group. The experimental protocol was approved by the local Animal Protection Ethical Committee of the Latvian Food and Veterinary Service (permission no. 55/15.03.2013.).

Mice were injected subcutaneously with 25 µg of protein in PBS formulated with 250 µg of Alhydrogel (Brenntag Biosector, Denmark) in a total volume of 0.2 mL (for normalisation using PBS). Animals were immunised on days 0, 14, 28 and bled for an ELISA test on day 42. As control groups were mice immunized with Alhydrogel alone and unstimulated in vivo mice.

The results of ELISA test (Table 1) suggest that the immune response against BB0689 is strongly enhanced, when protein is chemically coupled to VLPs.

**Table 1. ELISA tests of obtained sera.**

| Protein for immunization | Anti-Qβ antibody titer | Anti-BB0689 antibody titer |
|---|---|---|
| BB0689-Qβ | 1 : 12 000 | 1 : 54 000 |
| BB0689 | - | 1 : 3 000 |

### References

[1] Stricker RB, Johnson L (2014). Lyme disease: call for a "manhattan project" to combat the epidemic. PLoS Pathog. 10:e1003796.
[2] Cairns V, Godwin J (2005). Post-Lyme borreliosis syndrome: meta-analysis ofreported symptoms. Int J Epidemiol. 34:1340-5.
[3] Poland A.G. (2011). Vaccines against Lyme Disease: What Happened and What Lessons Can We Learn? Clinical Infectious Diseases; 52(S3):S253-S258
[4] Wressnigg N, Pöllabauer EM, Aichinger G, et al (2013). Safety and immunogenicity of a novel multivalent OspA vaccine against Lyme borreliosis in healthy adults: a double-blind, randomised, dose-escalation phase 1/2 trial. Lancet Infect Dis. 13:680-9.
[5] Barrett PN, Portsmouth D. (2013). The need for a new vaccine against lyme borreliosis. Expert Rev Vaccines. 12:101-103.
Brooks CS, Vuppala S.R., Jett AM, Akins DR (2006). Identification of Borrelia burgdorferi outer surface proteins. Infect Immune 74(1): 296-304.
Jennings GT, Bachmann MF (2008). The coming of age of virus-like particle vaccines. Biol. Chem. 389:521-536.
Freivalds J, Dislers A, Ose V, Skrastina D, Cielens I, Pumpens P, Sasnauskas K, Kazaks A. (2006). Assembly of bacteriophage Qbeta virus-like particles in yeast Saccharomyces cerevisiae and Pichia pastoris. J Biotechnol. 123(3):297-303.

Sequence listing
SEQ ID NO: 1
TYPE: nucleic acid
LENGTH: 33 bases
ORGANISM: artificial sequence
TOPOLOGY: linear
MOLECULAR TYPE: synthetic oligonucleotide
OTHER INFORMATION: BB0689fv primer

SEQUENCE: 1
catgccatgg gccataaaat agatacaaaa gaa 33

SEQ ID NO: 2
TYPE: nucleic acid
LENGTH: 42 bases
ORGANISM: artificial sequence
TOPOLOGY: linear
MOLECULAR TYPE: synthetic oligonucleotide
OTHER INFORMATION: BB0689rev primer

SEQUENCE: 2

gagtgcggcc gcttagcatc cagatccatt cttatatttt ct 42

SEQ ID NO: 3
TYPE: nucleic acid
LENGTH: 5732 bases
ORGANISM: artificial sequence
TOPOLOGY: circular
MOLECULAR TYPE: plasmid
OTHER INFORMATION: BB0689 expression plasmid

atccggatat agttcctcct ttcagcaaaa aacccctcaa gacccgttta gaggccccaa 1
ggggttatgc tagttattgc tcagcggtgg cagcagccaa ctcagcttcc tttcgggctt 61
tgttagcagc cggatctcag tggtggtggt ggtggtgctc gagtgcggcc gcttaattct 121
tatattttct ttttccaaaa agaactacaa atatatctat attgtcagtc gtttttaatc 181
tatagccacc tattttatcg gtatctgtat taataagagc ttctttgtgg cttggactat 241
taagccatgc attaactact ctgttaagtt caattcctga tgccagtatt tctcttgtta 301
aattaaagga ttgatcgtat ttatgtattc tttgcattgg ggttgtgcca aaaagggtgt 361
gagttattgt tctattttct cccagtttaa tggcatattc ttttgcaact ttttcaaggg 421
tatcatctat ttctagatgg tttagattta atttttttct caattcagca atttctgaat 481
atagaatttt catatcttct tttgtatcta ttttatggcc catggcgccc tgaaaataaa 541
gattctcagt agtggggatg tcgtaatcgc tcatggggtg atggtgatgg tgatgtttca 601
tggtatatct ccttcttaaa gttaaatcaa aattatttct agaggggaat tgttatccgc 661
tcacaattcc cctatagtga gtcgtattaa tttcgcggga tcgagatctc gatcctctac 721
gccggacgca tcgtggccgg catcaccggc gccacaggtg cggttgctgg cgcctatatc 781
gccgacatca ccgatgggga agatcgggct cgccacttcg ggctcatgag cgcttgtttc 841
ggcgtgggta tggtggcagg ccccgtggcc gggggactgt tgggcgccat ctccttgcat 901
gcaccattcc ttgcggcggc ggtgctcaac ggcctcaacc tactactggg ctgcttccta 961
atgcaggagt cgcataaggg agagcgtcga gatcccggac accatcgaat ggcgcaaaac 1021
ctttcgcggt atggcatgat agcgcccgga agagagtcaa ttcagggtgg tgaatgtgaa 1081
accagtaacg ttatacgatg tcgcagagta tgccggtgtc tcttatcaga ccgtttcccg 1141
cgtggtgaac caggccagcc acgtttctgc gaaaacgcgg gaaaaagtgg aagcggcgat 1201
ggcggagctg aattacattc ccaaccgcgt ggcacaacaa ctggcgggca aacagtcgtt 1261
gctgattggc gttgccacct ccagtctggc cctgcacgcg ccgtcgcaaa ttgtcgcggc 1321
gattaaatct cgcgccgatc aactgggtgc cagcgtggtg gtgtcgatgg tagaacgaag 1381
cggcgtcgaa gcctgtaaag cggcggtgca caatcttctc gcgcaacgcg tcagtgggct 1441
gatcattaac tatccgctgg atgaccagga tgccattgct gtggaagctg cctgcactaa 1501
tgttccggcg ttatttcttg atgtctctga ccagacaccc atcaacagta ttattttctc 1561
ccatgaagac ggtacgcgac tgggcgtgga gcatctggtc gcattgggtc accagcaaat 1621
cgcgctgtta gcgggcccat taagttctgt ctcggcgcgt ctgcgtctgg ctggctggca 1681

taaatatctc actcgcaatc aaattcagcc gatagcggaa cgggaaggcg actggagtgc 1741
catgtccggt tttcaacaaa ccatgcaaat gctgaatgag ggcatcgttc ccactgcgat 1801
gctggttgcc aacgatcaga tggcgctggg cgcaatgcgc gccattaccg agtccgggct 1861
gcgcgttggt gcggatatct cggtagtggg atacgacgat accgaagaca gctcatgtta 1921
tatcccgccg ttaaccacca tcaaacagga ttttcgcctg ctggggcaaa ccagcgtgga 1981
ccgcttgctg caactctctc agggccaggc ggtgaagggc aatcagctgt tgcccgtctc 2041
actggtgaaa agaaaaacca ccctggcgcc caatacgcaa accgcctctc cccgcgcgtt 2101
ggccgattca ttaatgcagc tggcacgaca ggtttcccga ctggaaagcg ggcagtgagc 2161
gcaacgcaat taatgtaagt tagctcactc attaggcacc gggatctcga ccgatgccct 2221
tgagagcctt caacccagtc agctccttcc ggtgggcgcg gggcatgact atcgtcgccg 2281
cacttatgac tgtcttcttt atcatgcaac tcgtaggaca ggtgccggca gcgctctggg 2341
tcattttcgg cgaggaccgc tttcgctgga gcgcgacgat gatcggcctg tcgcttgcgg 2401
tattcggaat cttgcacgcc ctcgctcaag ccttcgtcac tggtcccgcc accaaacgtt 2461
tcggcgagaa gcaggccatt atcgccggca tggcggcccc acgggtgcgc atgatcgtgc 2521
tcctgtcgtt gaggacccgg ctaggctggc ggggttgcct tactggttag cagaatgaat 2581
caccgatacg cgagcgaacg tgaagcgact gctgctgcaa aacgtctgcg acctgagcaa 2641
caacatgaat ggtcttcggt ttccgtgttt cgtaaagtct ggaaacgcgg aagtcagcgc 2701
cctgcaccat tatgttccgg atctgcatcg caggatgctg ctggctaccc tgtggaacac 2761
ctacatctgt attaacgaag cgctggcatt gaccctgagt gatttttctc tggtcccgcc 2821
gcatccatac cgccagttgt ttaccctcac aacgttccag taaccgggca tgttcatcat 2881
cagtaacccg tatcgtgagc atcctctctc gtttcatcgg tatcattacc cccatgaaca 2941
gaaatccccc ttacacggag gcatcagtga ccaaacagga aaaaaccgcc cttaacatgg 3001
cccgctttat cagaagccag acattaacgc ttctggagaa actcaacgag ctggacgcgg 3061
atgaacaggc agacatctgt gaatcgcttc acgaccacgc tgatgagctt taccgcagct 3121
gcctcgcgcg tttcggtgat gacggtgaaa acctctgaca catgcagctc ccggagacgg 3181
tcacagcttg tctgtaagcg gatgccggga gcagacaagc ccgtcagggc gcgtcagcgg 3241
gtgttggcgg gtgtcggggc gcagccatga cccagtcacg tagcgatagc ggagtgtata 3301
ctggcttaac tatgcggcat cagagcagat tgtactgaga gtgcaccata tatgcggtgt 3361
gaaataccgc acagatgcgt aaggagaaaa taccgcatca ggcgctcttc cgcttcctcg 3421
ctcactgact cgctgcgctc ggtcgttcgg ctgcggcgag cggtatcagc tcactcaaag 3481
gcggtaatac ggttatccac agaatcaggg gataacgcag gaaagaacat gtgagcaaaa 3541
ggccagcaaa aggccaggaa ccgtaaaaag gccgcgttgc tggcgttttt ccataggctc 3601
cgcccccctg acgagcatca caaaaatcga cgctcaagtc agaggtggcg aaacccgaca 3661
ggactataaa gataccaggc gtttccccct ggaagctccc tcgtgcgctc tcctgttccg 3721
accctgccgc ttaccggata cctgtccgcc tttctccctt cgggaagcgt ggcgctttct 3781
catagctcac gctgtaggta tctcagttcg gtgtaggtcg ttcgctccaa gctgggctgt 3841
gtgcacgaac cccccgttca gcccgaccgc tgcgccttat ccggtaacta tcgtcttgag 3901
tccaacccgg taagacacga cttatcgcca ctggcagcag ccactggtaa caggattagc 3961
agagcgaggt atgtaggcgg tgctacagag ttcttgaagt ggtggcctaa ctacggctac 4021
actagaagga cagtatttgg tatctgcgct ctgctgaagc cagttacctt cggaaaaaga 4081
gttggtagct cttgatccgg caaacaaacc accgctggta gcggtggttt ttttgtttgc 4141
aagcagcaga ttacgcgcag aaaaaaagga tctcaagaag atcctttgat cttttctacg 4201
gggtctgacg ctcagtggaa cgaaaactca cgttaaggga ttttggtcat gaacaataaa 4261
actgtctgct tacataaaca gtaatacaag gggtgttatg agccatattc aacgggaaac 4321
gtcttgctct aggccgcgat taaattccaa catggatgct gatttatatg ggtataaatg 4381
ggctcgcgat aatgtcgggc aatcaggtgc gacaatctat cgattgtatg ggaagcccga 4441
tgcgccagag ttgtttctga aacatggcaa aggtagcgtt gccaatgatg ttacagatga 4501
gatggtcaga ctaaactggc tgacggaatt tatgcctctt ccgaccatca agcattttat 4561
ccgtactcct gatgatgcat ggttactcac cactgcgatc cccgggaaaa cagcattcca 4621
ggtattagaa gaatatcctg attcaggtga aaatattgtt gatgcgctgg cagtgttcct 4681
gcgccggttg cattcgattc ctgtttgtaa ttgtcctttt aacagcgatc gcgtatttcg 4741
tctcgctcag gcgcaatcac gaatgaataa cggtttggtt gatgcgagtg attttgatga 4801
cgagcgtaat ggctggcctg ttgaacaagt ctggaaagaa atgcataaac ttttgccatt 4861
ctcaccggat tcagtcgtca ctcatggtga tttctcactt gataacctta tttttgacga 4921
ggggaaatta ataggttgta ttgatgttgg acgagtcgga atcgcagacc gataccagga 4981
tcttgccatc ctatggaact gcctcggtga gttttctcct tcattacaga aacggctttt 5041
tcaaaaatat ggtattgata atcctgatat gaataaattg cagtttcatt tgatgctcga 5101
tgagtttttc taagaattaa ttcatgagcg gatacatatt tgaatgtatt tagaaaaata 5161
aacaaatagg ggttccgcgc acatttcccc gaaaagtgcc acctgaaatt gtaaacgtta 5221
atattttgtt aaaattcgcg ttaaattttt gttaaatcag ctcatttttt aaccaatagg 5281
ccgaaatcgg caaaatccct tataaatcaa aagaatagac cgagataggg ttgagtgttg 5341
ttccagtttg gaacaagagt ccactattaa agaacgtgga ctccaacgtc aaagggcgaa 5401
aaaccgtcta tcagggcgat ggcccactac gtgaaccatc accctaatca agttttttgg 5461

ggtcgaggtg ccgtaaagca ctaaatcgga accctaaagg gagcccccga tttagagctt 5521
gacggggaaa gccggcgaac gtggcgagaa aggaagggaa gaaagcgaaa ggagcgggcg 5581
ctagggcgct ggcaagtgta gcggtcacgc tgcgcgtaac caccacaccc gccgcgctta 5641
atgcgccgct acagggcgcg tcccattcgc ca 5701

SEQ ID NO: 4
TYPE: protein
LENGTH: 163 amino acids
ORGANISM: Borrelia burgdorferi B31
TOPOLOGY: linear
MOLECULAR TYPE: Outer surface protein

SEQUENCE:4
Met Lys His His His His His His Pro Met Ser Asp Tyr Asp Ile
                5                   10                  15
Pro Thr Thr Glu Asn Leu Tyr Phe Gln Gly Ala Met Gly His Lys
                20                  25                  30
Ile Asp Thr Lys Glu Asp Met Lys Ile Leu Tyr Ser Glu Ile Ala
                35                  40                  45
Glu Leu Arg Lys Lys Leu Asn Leu Asn His Leu Glu Ile Asp Asp
                50                  55                  60
Thr Leu Glu Lys Val Ala Lys Glu Tyr Ala Ile Lys Leu Gly Glu
                65                  70                  75
Asn Arg Thr Ile Thr His Thr Leu Phe Gly Thr Thr Pro Met Gln
                80                  85                  90
Arg Ile His Lys Tyr Asp Gln Ser Phe Asn Leu Thr Arg Glu Ile
                95                  100                 105
Leu Ala Ser Gly Ile Glu Leu Asn Arg Val Val Asn Ala Trp Leu
                110                 115                 120
Asn Ser Pro Ser His Lys Glu Ala Leu Ile Asn Thr Asp Thr Asp
                125                 130                 135
Lys Ile Gly Gly Tyr Arg Leu Lys Thr Thr Asp Asn Ile Asp Ile
                140                 145                 150
Phe Val Val Leu Phe Gly Lys Arg Lys Tyr Lys Asn Cys
                155                 160

SEQ ID NO: 5
TYPE: protein
LENGTH: 139 amino acids
ORGANISM: Borrelia burgdorferi B31
TOPOLOGY: linear
MOLECULAR TYPE: Outer surface protein

SEQUENCE:5
Gly Ala Met Gly His Lys Ile Asp Thr Lys Glu Asp Met Lys Ile
                5                   10                  15
Leu Tyr Ser Glu Ile Ala Glu Leu Arg Lys Lys Leu Asn Leu Asn
                20                  25                  30
His Leu Glu Ile Asp Asp Thr Leu Glu Lys Val Ala Lys Glu Tyr
                35                  40                  45
Ala Ile Lys Leu Gly Glu Asn Arg Thr Ile Thr His Thr Leu Phe
                50                  55                  60
Gly Thr Thr Pro Met Gln Arg Ile His Lys Tyr Asp Gln Ser Phe
                65                  70                  75
Asn Leu Thr Arg Glu Ile Leu Ala Ser Gly Ile Glu Leu Asn Arg
                80                  85                  90
Val Val Asn Ala Trp Leu Asn Ser Pro Ser His Lys Glu Ala Leu
                95                  100                 105
Ile Asn Thr Asp Thr Asp Lys Ile Gly Gly Tyr Arg Leu Lys Thr
                110                 115                 120
Thr Asp Asn Ile Asp Ile Phe Val Val Leu Phe Gly Lys Arg Lys

                125                 130                 135
Tyr Lys Asn Cys

## Claims

1. A protein, comprising amino acids identical at least 80% to modified *Borrelia burgdorferi* BB0689 protein (as set forth in SEQ ID 5), chemically coupled to bacteriophage Qβ virus-like particles.

2. Composition which comprises a protein, according to Claim 1, chemically coupled to bacteriophage Qβ virus-like particles.

3. Use of a composition according to Claims 2, for the manufacture of a vaccine against Lyme disease.

4. Composition according Claim 2, for use as a therapeutic anti-Lyme vaccine.

5. A method for the preparation of immunogenic composition for the treatment of Lyme disease, wherein method comprises:
a) preparation and purification of protein, comprising amino acids identical at least 80% to modified Borrelia burgdorferi BB0689 protein as set forth in SEQ ID 5;
b) providing a purified bacteriophage Qβ VLPs;
c) coupling of protein of step a) to bacteriophage Qβ virus-like particles from step b).
